(19) Europäisches Patentamt
European Patent Office
Office européen des brevets

(11) **EP 4 272 677 A2**

(12) **EUROPÄISCHE PATENTANMELDUNG**

(43) Veröffentlichungstag:
**08.11.2023  Patentblatt 2023/45**

(21) Anmeldenummer: **23189494.0**

(22) Anmeldetag: **07.07.2020**

(51) Internationale Patentklassifikation (IPC):
***A61B 18/26*** *(2006.01)*

(52) Gemeinsame Patentklassifikation (CPC):
**A61B 18/26; A61B 1/00006; A61B 1/12;
A61B 1/128; A61B 1/307;** A61B 18/22;
A61B 2018/00642; A61B 2018/0066;
A61B 2018/00678; A61B 2018/00791;
A61B 2018/00863; A61B 2018/263

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **11.07.2019  DE 202019103823 U**

(62) Dokumentnummer(n) der früheren Anmeldung(en)
nach Art. 76 EPÜ:
**20739315.8 / 3 996 619**

(27) Früher eingereichte Anmeldung:
**07.07.2020 PCT/EP2020/069125**

(71) Anmelder: **Albert-Ludwigs-Universität Freiburg
79098 Freiburg (DE)**

(72) Erfinder:
• **MIERNIK, Arkadiusz
79098 Freiburg (DE)**
• **HEIN, Simon
79098 Freiburg (DE)**
• **PETZOLD, Ralf
79098 Freiburg (DE)**

(74) Vertreter: **Rösler, Uwe
Rösler Patentanwaltskanzlei
Landsberger Strasse 480a
81241 München (DE)**

Bemerkungen:
•Diese Anmeldung ist am 03.08.2023 als
Teilanmeldung zu der unter INID-Code 62 erwähnten
Anmeldung eingereicht worden.
•Claims filed after the date of filing of the application
/ after the date of receipt of the divisional application
(Rule 68(4) EPC).

(54) **SYSTEM ZUR TEMPERATURÜBERWACHUNG BEI DER DURCHFÜHRUNG EINER LASERLICHT-BASIERTEN INTRAKORPORALEN LITHOTRIPSIE**

(57)    Beschrieben wird ein System zur Temperaturüberwachung bei der Durchführung einer laserlichtbasierten Lithotripsie, bei der eine Endoskopanordnung zum Einsatz kommt, die einen Arbeitskanal für einen Lichtleiter, der proximalseitig mit einem Laser optisch gekoppelt ist und distalseitig eine Lichtaustrittsapertur aufweist, sowie einen Spülflüssigkeitskanal umfasst, der im Bereich der Lichtaustrittsapertur distalseitig offen mündet und proximalseitig mit einem Spülflüssigkeitsreservoir fluidisch verbunden ist.

Das System zeichnet sich durch eine Moduleinheit aus, die folgende Komponenten umfasst:
- Durchflusssenor, der längs des Spülflüssigkeitskanals anbringbar ist und kontaktfrei zur Spülflüssigkeit Spülflussrate bestimmt,
- Eingabemittel, über das Betriebsparameter des Lasers, auf deren Grundlage eine am Ort der Lichtaustrittapertur applizierbare Laserleistung ermittelbar ist, einer mit dem Eingabemittel verbundenen Rechnereinheit übermittelbar sind,
- Temperatursensor, der längs des Spülflüssigkeitskanals anbringbar ist und kontaktfrei zur Spülflüssigkeit eine Temperatur der Spülflüssigkeit bestimmt,
- eine in der Rechnereinheit implementierte Auswerteinheit, die zumindest auf der Grundlage der Spülflussrate, der Temperatur der Spülflüssigkeit (Ts) sowie der ermittelten applizierten Laserleistung eine sich während der laserlichtbasierten Lithotripsie intrakorporal am Ort der Lichtaustrittapertur ausbildende Temperatur T numerisch ermittelt, sowie
- Komparatoreinheit, die die ermittelte Temperatur T mit einem Schwellwert vergleicht und bei Überschreiten des Schwellwertes ein Signal erzeugt.

EP 4 272 677 A2

**Beschreibung**

**Technisches Gebiet**

Die Erfindung bezieht sich auf ein System zur Temperaturüberwachung bei der Durchführung einer laserlichtbasierten intrakorporalen Lithotripsie, bei der eine Endoskopanordnung zum Einsatz kommt, die einen Arbeitskanal für einen Lichtleiter, der proximalseitig mit einem Laser optisch gekoppelt ist und distalseitig eine Lichtaustrittsapertur aufweist, sowie einen Spülflüssigkeitskanal umfasst, der im Bereich der Lichtaustrittsapertur distalseitig offen mündet und proximalseitig mit einem Spülflüssigkeitsreservoir fluidisch verbunden ist.

**Stand der Technik**

Die intrakorporale Lithotripsie betrifft eine minimalinvasive chirurgische Methode zur Zertrümmerung von Steinkonkrementen, beispielsweise Gallen-, Harn- oder Nierensteine, die sich vornehmlich in Ausführungsgängen der betreffenden Organe ansammeln und starke Schmerzen sowie andere medizinischen Probleme verursachen können. Unter den diversen bekannten intrakorporalen Lithotripsieverfahren hat sich die laserinduzierte Lithotripsie etabliert, bei der energiereiche Laserstrahlung über eine Lichtleitfaser an den Ort eines zu zertrümmernden Steines in Form von Laserpulsen appliziert wird. Durch Wechselwirkung der Laserpulse mit dem intrakorporalen Medium bildet sich eine Dampfblase aus, durch deren pulsartige Ausdehnung Druckwellen entstehen, die zur Fragmentation des Steins führen. Die bei der laserinduzierten Lithotripsie entstehenden Steinfragmente können in Abhängigkeit ihrer Größe auf natürlichem Wege ausgeschieden oder mit geeignet ausgebildeten endoskopischen Greifwerkzeugen extrahiert werden.

Zur Durchführung der laserbasierten Lithotripsie kommen Endoskope zum Einsatz, die wenigstens einen Arbeitskanal für einen Lichtleiter vorsehen, der proximalseitig mit einem Laser optisch gekoppelt ist und distalseitig eine Lichtaustrittsapertur aufweist. Um den organischen Zugängen zu den Steinen mit dem Endoskop folgen zu können und den Patienten während der Behandlung möglichst wenig zu belasten, können derartige Endoskope biegsam und flexibel gestaltet werden. Zudem kann man über derartige Endoskop eine Spülflüssigkeit meistens über einen Arbeitskanal applizieren, der im Bereich der Lichtaustrittsapertur distalseitig mündet und proximalseitig mit einer Spülflüssigkeitsfördereinrichtung, vorzugsweise in Form einer Spülspritze und/oder einer steuer- oder regelbaren Förderpumpe, sowie einem Spülflüssigkeitsreservoir fluidisch verbunden ist.

Mit Hilfe einer derartigen Endoskopanordnung, umfassend das Endoskop und die daran angeschlossenen, vorstehend erwähnten Peripheriegeräte, gilt es die intrakorporal am Ort des zu zertrümmernden Steins applizierte Laserleistung sowie die Menge der Spülflüssigkeit, die distalseits zum Spülkanal ausgetragen wird, derart aufeinander abzustimmen, dass zum einen eine effektive Steinzertrümmerung erfolgen kann, und zum anderen keine laserlichtbedingte lokale Überhitzung von umliegenden Gewebebereichen auftritt, die zu irreversiblen Gewebeschädigungen führen kann. Zumeist verfügen derartige Endoskope über eine zusätzliche Glasfaseroptik für den Operateur oder einen bildgebenden Sensor an der Endoskopspitze, den es mit Hilfe einer dosierten Spülflüssigkeitszuführung optisch freizuhalten gilt, um stets einen freien Blick für den Operateur zur Überwachung des Zertrümmerungsprozesses zu gewährleisten.

Die Druckschrift US 2018/0055568 A1 beschreibt eine gattungsgemäße Endoskopanordnung zur Durchführung einer laserbasierten Lithotripsie, deren zugeordnete Spülflüssigkeitsfördereinheit in Abhängigkeit wenigstens eines den Lithotripsieprozess beeinflussenden Parameters regelbar ist, d.h. die Spülrate wird in Abhängigkeit des Lithotripsieprozesses beeinflusst. Neben einer optischen Überwachung des Zertrümmerungsvorganges, sieht die bekannte Endoskopanordnung einen am distalen Ende des Endoskops angebrachten Temperatursensor vor, der die distalseitige Temperatur der ihn umgebenden Flüssigkeitzu erfassen vermag und die der Spülflüssigkeitsfördereinheit zur Einstellung der Spülflüssigkeitsrate zugrunde gelegt wird.

Auch bei dem bekannten medizinischen Endoskop gemäß der Druckschrift DE 20 2017 102 316 U1, das zur Durchführung einer laserbasierten Lithotripsie geeignet ist, ist zu Zwecken einer Temperaturmessung ein Umgebungstemperatursensor am distalen Bereich des Endoskops angeordnet.

Derartige mit Sensoren ausgestattete Endoskopanordnungen stellen hochkomplexe medizinische Instrumente dar, die einer langwierigen und umfangreichen medizinisch technischen Zulassungsprüfung unterliegen.

**Darstellung der Erfindung**

Der Erfindung liegt die Aufgabe zugrunde, ein System zur Temperaturüberwachung bei der Durchführung einer laserlichtbasierten intrakorporalen Lithotripsie, bei der eine Endoskopanordnung zum Einsatz kommt, die einen Arbeitskanal für einen Lichtleiter, der proximalseitig mit einem Laser optisch gekoppelt ist und distalseitig eine Lichtaustrittsapertur aufweist, sowie einen Spülflüssigkeitskanal umfasst, der im Bereich der Lichtaustrittsapertur distalseitig mündet und proximalseitig mit einem Spülflüssigkeitsreservoir fluidisch verbunden ist, derart weiterzubilden, so dass eine überwachte intrakorporale Lithotripsiedurchführung auch mit gattungsgemäßen Standard Endoskopen möglich wird, ohne geld- und zeitraubende Aufwendungen leisten zu müssen, die in Zusammenhang mit einer medizinisch technischen Zulassungsprüfung bzw. Zertifizierung verbunden sind. Es gilt nach Maßnahmen zu suchen, mit denen bereits im Einsatz befindliche Endoskope, Lasersysteme und weitere medizinisch-technische Vorrichtungen, wie beispielsweise Spülflüssigkeitsvorrichtungen zur passiven und aktiven Spülung die zur laserbasierten Lithotripsie grundsätzlich geeignet ausgebildet sind, mit geeignet gewählten Komponenten nachzurüsten, um mögliche thermisch induzierte Gewebedegradationen im unmittelbaren Umfeld am Ort der Steinzertrümmerung sicher ausschließen zu können.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie der weiteren Beschreibung insbesondere unter Bezugnahme auf die Ausführungsbeispiele zu entnehmen.

Generell ist die intrakorporale Laserapplikation ohne Spülung, d.h. ohne jegliche Spülflüssigkeitsversorgung am Bereich der distalseitigen Lichtaustrittsapertur des Lichtleiters, nur kurzfristig, d.h. innerhalb weniger Sekunden möglich, zumal ohne jegliche spülungsbedingte Kühlung eine kritische Temperatur von ca. 42°C erreicht und überschritten wird, ab der das umliegende Gewebe thermisch irreversibel geschädigt wird. Demzufolge ist eine Spülung während der laserlichtbasierten Lithotripsie unverzichtbar, um eine ausreichende Kühlung zu gewährleisten.

Anhand einer großen Vielzahl durchgeführter Versuche zur laserbasierten Lithotripsie konnte ein belastbarer, numerischer bzw. theoretischer Zusammenhang zwischen der intrakorporal applizierten Laserleistung, der Spülflüssigkeitsrate, der Temperatur der Spülflüssigkeit und einer sich intrakorporal während der Laserpulsapplikation einstellenden Temperatur gefunden werden.

Auf der Basis dieser Erkenntnis eröffnet sich die Möglichkeit, an bekannte Endoskope mit wenigstens einem Arbeitskanal für einen Lichtleiter, der proximalseitig mit einem Laser optisch gekoppelt ist und distalseitig eine Lichtaustrittsapertur aufweist sowie einen Spülflüssigkeitskanal umfasst, der getrennt zum oder einheitlich mit dem Arbeitskanal ausgebildet sein kann, und im Bereich der Lichtaustrittsapertur distalseitig offen mündet und proximalseitig mit einer optionalen Spülflüssigkeitsfördereinheit und einem Spülflüssigkeitsreservoir fluidisch verbunden ist, mit einer lösungsgemäß ausgebildeten Moduleinheit in der folgenden Weise zu kombinieren: Die Moduleinheit umfasst wenigstens einen Durchflusssensor sowie wenigstens einen Temperatursensor, die jeweils längs des Spülflüssigkeitskanals und/oder einer mit dem Spülflüssigkeitskanal fluidisch verbundenen Zuleitung, vorzugsweise lösbar fest, längs des extrakorporalen Bereich der Endoskopanordnung anbringbar sind. Der Durchflusssensor ist derart ausgebildet und angeordnet, dass die Spülflussrate kontaktfrei zur Spülflüssigkeit ermittelbar ist. Gleichsam ist der Temperatursensor derart ausgebildet und längs des Spülflüssigkeitskanals und/oder der mit dem Spülflüssigkeitskanal fluidisch verbundenen Zuleitung angebracht, dass die Spülflüssigkeitstemperatur kontaktfrei ermittelbar ist.

Ferner umfasst die Moduleinheit entweder ein Eingabemittel, über das Betriebsparameter des Lasers eingebbar und einer mit dem Eingabemittel verbundenen Rechnereinheit übermittelbar sind, oder eine Schnittstelle, über welche die Betriebsparameter des Lasers direkt übertragen werden können. Die Betriebsparameter des Lasers betreffen vorzugsweise die Laserleistung, wobei über die Laserpulsfrequenz sowie die Einzelpulsenergie die Berechnung einer am Ort der Lichtaustrittsapertur applizierten Laserleistung möglich ist.

Innerhalb der Rechnereinheit ist zudem eine Auswerteeinheit implementiert, die auf der Grundlage der sensorisch erfassten Spülflussrate, der sensorisch erfassten Temperatur der Spülflüssigkeit sowie der ermittelten intrakorporal applizierten Laserleistung eine sich während der laserlichtbasierten Lithotripsie intrakorporal am Ort der Lichtaustrittsapertur ausbildende Temperatur numerisch ermittelt. Getrennt zur oder integriert in der Rechnereinheit ist eine Komparatoreinheit angeordnet, die die ermittelte Temperatur mit einem einstellbaren Schwellwert vergleicht, bspw. 42 °C, und bei Überschreiten des Schwellwertes ein Signal erzeugt.

In einer bevorzugten Ausführungsform ist zudem eine Signaleinheit vorgesehen, die kabellos oder kabelgebunden mit der Komparatoreinheit verbunden ist und durch das von der Komparatoreinheit erzeugte Signal haptisch, akustisch und/oder visuell wahrnehmbar in Erscheinung zu treten vermag. Durch die Signaleinheit wird der die Lithotripsie durchführende Arzt vor möglichen intrakorporalen Überhitzungseffekten gewarnt, um auf diese Weise die Lithotripsiebehandlung zu unterbrechen oder anderweitige Maßnahmen zu treffen.

Die lösungsgemäße, vorzugsweise als Nachrüstsatz oder Nachrüstkit ausgebildete Moduleinheit umfasst ausschließlich Komponenten, die modulartig an eine im Einsatz befindliche Endoskopanordnung anbringbar sind, ohne die grundsätzliche Funktionalität der Endoskopanordnung zu beeinträchtigen. Das lösungsgemäße System stellt somit ein den Arzt bei der Durchführung einer laserbasierten Lithotripsie unterstützendes Sicherheitssystem dar, über das der Arzt zuverlässige und belastbare Informationen erhält, um den Lithotripsieprozess effektiv und patientenschonend durchführen zu können.

Die einzigen hierfür erforderlichen Informationen betreffen die aktuelle Temperatur der über die Endoskopanordnung vermittels des Arbeitskanals oder des Spülflüssigkeitskanals intrakorporal eingebrachte Spülflüssigkeit, die am Ort des zu zertrümmernden Steines applizierte Laserleistung sowie die Spülrate, mit der die Spülflüssigkeit durch den Arbeitskanal oder Spülflüssigkeitskanal intrakorporal am Ort der Laserlichtapplikation eingebracht wird.

Die für die Spülflüssigkeitstemperatur sowie Spülrate erforderlichen Sensoren stellen jeweils lösbar fest an den Spülflüssigkeitskanal und/oder an die mit dem Spülflüssigkeitskanal fluidisch verbundenen Zuleitung anbringbare Bauelemente dar, die in einem Bereich der Endoskopanordnung angebracht sind, der nicht intrakorporal in den Körper eingebracht wird. So eignet sich in bevorzugter Weise als ein die Spülflussrate bestimmender Sensor ein Durchflusssensor in Art eines Ultraschallsensors. Zur Erfassung der Spülflüssigkeitstemperatur eignet sich ein Temperatursensor in Art eines Infrarotsensors.

Zur Ermittlung der sich am Ort der Lichtaustrittsapertur des Lichtleiters ausbildenden Temperatur sind die am Laser vorgebbaren Betriebsparameter betreffend die Laserpulsfrequenz sowie die Einzelpulsenergie erforderlich. Diese Betriebsparameter sind über ein geeignet ausgebildetes Eingabemittel, beispielsweise per Tastatur oder mittels eines per Sprachsteuerung arbeitenden Eingabemittels oder über eine direkte Schnittstelle am Lasergerät der Rechnereinheit zuführbar.

Die innerhalb der Rechnereinheit implementierte Auswerteeinheit bestimmt auf der Grundlage der nachfolgenden Formel die sich am Ort der Lichtaustrittsapertur ausbildende Temperatur T in der folgenden Weise:

$$T = \left( 14{,}4\,K * \frac{[Laserleistung\,[W]]}{Sp\ddot{u}lflussrate\,\left[\frac{ml}{min}\right]} \right) + Temp.\,d.\,Sp\ddot{u}lfl\ddot{u}ssigkeit\,[K]$$

Die numerische Abhängigkeit der sich während einer laserbasierten Lithotripsie intrakorporal am Ort der Laserlichtapplikation ausbildende Temperatur von der applizierten Laserleistung, der Spülflussrate sowie der Temperatur der Spülflüssigkeit ist in einer großen Vielzahl von in vitro als auch ex vivo durchgeführten Untersuchungen bestätigt worden.

Gleichwohl der vorstehende Algorithmus ein vereinfachter Formelzusammenhang darstellt, lassen sich damit die während der laserbasierten Lithotripsie zu erwartenden intrakorporalen Endtemperaturen am Ort der Steinzertrümmerung hinreichend genau voraussagen, sodass lokale Überhitzungen, die zu irreversiblen Gewebeschädigungen im unmittelbaren Umfeld am Ort der laserbasierten Lithotripsie führen können, ausgeschlossen werden können.

Für den Fall, dass die Lasereinheit keine Schnittstelle besitzt, um den Laserbetrieb anzuzeigen, umfasst die Moduleinheit zusätzlich eine Mikrofoneinheit zur Erkennung eines den Laserbetrieb anzeigenden akustischen Signals, das der Auswerteeinheit zum Zwecke einer zeitsynchronen Signalauswertung zum tatsächlich stattfindenden Laserbetrieb übermittelt wird. So erzeugt der Laser während des Laserbetriebes eindeutig detektierbare akustische Signale, die mit der Mikrofoneinheit zeitaufgelöst erfassbar sind.

Gleichwohl der für die Durchführung der Lithotripsie verantwortliche Arzt der alleinige Entscheidungsträger bei der Durchführung der laserbasierten Lithotripsie ist, kann in einer weiteren Ausführungsform die lösungsgemäß ausgebildete Moduleinheit einen zusätzlichen Notausschalter umfassen, d.h. zusätzlich zu der akustisch, visuell und/oder haptisch in Erscheinung tretenden Signaleinheit, kann das Signal eine Unterbrechung der Stromzuführung zumindest für die Laserquelle auslösen.

In einer weiteren bevorzugten Ausführungsform weist die an eine Endoskopanordnung adaptierbare Moduleinheit zusätzlich eine Einheit zur Erfassung des Füllstandes der innerhalb des Spülflüssigkeitsreservoirs enthaltenen Menge an Spülflüssigkeit auf. Die Einheit ist als am Spülflüssigkeitsreservoir vorgesehener Sensor und/oder in Form eines in der Recheneinheit implementierten, softwarebasierten Auswertealgorithmus ausgebildet, der auf der Grundlage der sensoriell erfassten Spülflussrate und der gemessenen Zeitdauer, während der ein Spülflüssigkeitsaustrag erfolgt, den aktuellen Füllstand ermittelt. Das den Füllstand repräsentierende Sensorsignal bzw. der den Füllstand repräsentierende, numerisch ermittelte Füllstandswert dient zumindest zur Überwachung des Ist-Zustandes sowie einer Prädiktion einer Zeitdauer bis zur vollständigen Entleerung des Spülflüssigkeitsreservoirs oder der Warnung bei Erreichen einer Mindestrestmenge. Die hierdurch gewonnenen Informationen werden durch eine geeignete Anzeige dem behandelnden Arzt optisch oder akustisch wahrnehmbar dargestellt. Die Anzeige kann durch die Signaleinheit erfolgen, über die der die Lithotripsie durchführende Arzt vor möglichen intrakorporalen Überhitzungseffekten gewarnt wird, oder durch eine zusätzliche Anzeige.

Eine weitere Ausführungsform ermöglicht die zusätzliche Überwachung der Temperatur der zugeführten Spülflüssigkeit. Hierzu wird die sensoriell erfasste Temperatur der Spülflüssigkeit (Ts) vorzugsweise als alphanumerischer Wert auf einem Sichtgerät dargestellt. Ferner wird die gemessene Temperatur vorzugsweise der Komparatoreinheit oder einer weiteren Komparatoreinheit zugeführt, wobei bei Unter- oder Überschreiten der Temperatur der Spülflüssigkeit (Ts) unter oder über einen kritischen Temperaturwert ein Signal erzeugt wird, das als Warnung für den behandelnden Arzt dient, bspw. bei einer zu kalten Spülflüssigkeit erfolgt eine Warnung, um die Gefahr einer Unterkühlung für den Patienten zu vermeiden.

In einer weiteren möglichen Ausführungsform sieht die Spülflüssigkeitsfördereinheit ein Mittel vor, durch das die Spülflüssigkeitsrate in Abhängigkeit der sich am Ort der Lichtaustrittapertur ausbildenden Temperatur T und/oder wenigstens eines Betriebsparameters des Lasers regelbar ist. Auf diese Weise eröffnet sich die Möglichkeit einer aktiven Regelung der Spülflüssigkeitsrate der Spülflüssigkeitsfördereinheit in Form einer Pumpe in Abhängigkeit von der applizierten Laserlichtleistung und/oder der berechneten Temperaturen im Gebiet des Eingriffes.

Das lösungsgemäße System zur Temperaturüberwachung bei der Durchführung einer laserlichtbasierten Lithotripsie lässt sich für verschiedene anatomische Bereiche einsetzen, so bspw. im Blasen-, Ureter- oder Nierenbeckenbereich. Je nach Anwendungsbereich kann zwischen verschiedenen Modi zur Anpassung an den Eingriff gewählt werden. Dabei bietet das System die Möglichkeit der Wahl eines Prostata, Harnblasen-, Ureter- oder Nierenbeckenmodus, welche sich durch definierbare Warngrenzen und hinterlegte Kennlinien unterscheiden. Dadurch kann das individuelle Gefahrenspektrum der verschiedenen Operationen adressiert werden.

So lässt sich das System bei allen laserbasierten Verfahren in der Urologie eingesetzt werden, insbesondere bei der Laserlithotripsie, der lasergestützten Prostataenukleation (HoLEP, ThuLEP), der Laservaporisation (PVP) und der interstitiellen Laservaporisation (ILC).)

## Wege zur Ausführung der Erfindung, gewerbliche Verwendbarkeit

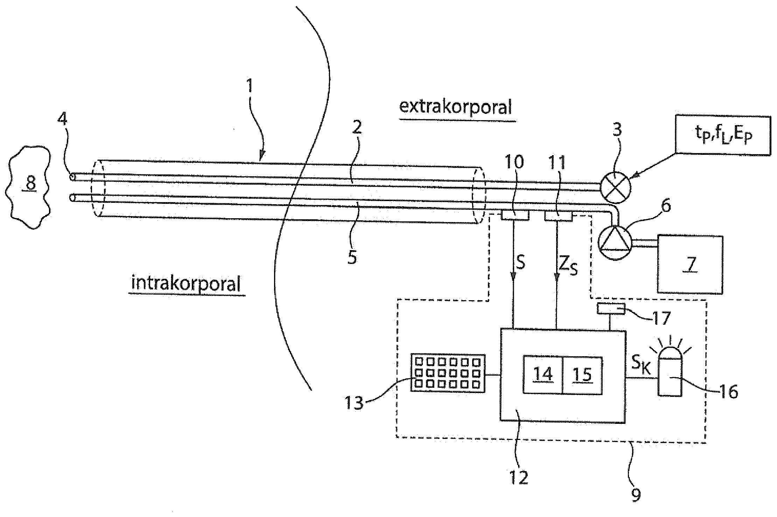

Die einzige Figur zeigt ein Endoskop 1 mit einem Arbeitskanal für einen Lichtleiter 2, der proximalseitig mit einem Laser 3 optisch gekoppelt ist und distalseitig eine Lichtaustrittsapertur 4 aufweist. Ferner verfügt das Endoskop 1 über einen Spülflüssigkeitskanal 5, der im Bereich der Lichtaustrittsapertur 4 distalseitig offen mündet und proximalseitig mit einer Flüssigkeitsfördereinheit 6 sowie einem Flüssigkeitsreservoir 7 fluidisch verbunden ist. Selbstverständlich sind auch Endoskope bekannt, welche den Spülflüssigkeitskanal 5 mit dem Arbeitskanal für den Lichtleiter 2 in einem Kanal vereinen. Derartige an sich bekannte Endoskopanordnungen dienen zur Zertrümmerung intrakorporaler Steine 8 im Wege der laserbasierten Lithotripsie.

Das lösungsgemäße System zur Temperaturüberwachung betrifft eine mit der Endoskopanordnung, umfassend das Endoskop 1, den Laser 3, die Flüssigkeitsfördereinheit 6 sowie das Flüssigkeitsreservoir 7, in dessen extrakorporalen Bereich kombinierbare Moduleinheit 9, die sich einer bevorzugten Ausführungsform aus den folgenden Komponenten zusammensetzt:

Längs der Spülflüssigkeitszuleitung 5 ist ein Durchflusssensor 10, vorzugsweise in Art eines Ultraschallsensors, lösbar fest angebracht. Ebenfalls längs der Spülflüssigkeitskanal 5 oder einer mit diesem fluidische verbundenen Zuleitung ist ein Temperatursensor 11, vorzugsweise in Form eines Infrarotsensors, zur Erfassung der Spülflüssigkeitstemperatur lösbar fest angebracht. Die mittels der Sensoren 10, 11 erzeugten Sensorsignale S, Ts werden einer Rechnereinheit 12 drahtgebunden oder drahtlos übermittelt.

Ferner ist ein Eingabemittel 13 vorgesehen, vorzugsweise in Art einer Schnittstelle zur Lasereinheit zur automatischen Betriebsparameterübergabe, alternativ in Art einer manuell bedienbaren Tastatur, über die die Betriebsparameter des Lasers 3 eingebbar sind. Für den Betrieb des Lasers 3 können die die Laserpulsfrequenz $f_L$ sowie die Einzelpulsenergie $E_P$ durch einen Bediener vorgegeben werden. Eben diese Betriebsparameter sind über das Eingabemittel 13 der Rechnereinheit 12 zuführbar, auf deren Grundlage die am Ort der Lichtaustrittsapertur 4 applizierbare Laserleistung errechnet wird.

Innerhalb der Rechnereinheit 12 ist eine Auswerteeinheit 14 implementiert, die auf der Grundlage der Betriebsparameter des Lasers, der sensorisch erfassten Spülflussrate S sowie der Temperatur Ts der Spülflüssigkeit die sich aktuell am Ort der Lichtaustrittsapertur 4 ausbildende Temperatur unter Zugrundelegung des nachfolgenden Algorithmus ermittelt:

$$T = \left( 14{,}4 \, K * \frac{[Laserleistung \, [W]]}{Sp\ddot{u}lflussrate \left[ \frac{ml}{min} \right]} \right) + Temp. \, d. \, Sp\ddot{u}lfl\ddot{u}ssigkeit \, [K]$$

Die ermittelte intrakorporale Temperatur T wird im Weiteren in einer Komparatoreinheit 15 mit einem einstellbaren Schwellwert verglichen, der vorzugsweise $T_K$= 42°C beträgt. Im Falle eines Überschreitens des Schwellwertes $T_K$ erzeugt die Komparatoreinheit 15 ein Signal $S_K$, das einer Signaleinheit 16 zugeführt wird, die für den die Lithotripsie durchführenden Arzt haptisch, akustisch und/oder visuell wahrnehmbar in Erscheinung tritt.

Zusätzlich ist eine Mikrofoneinheit 17 mit der Rechnereinheit 12 verbunden, mit der die Aktivität des Lasers 3 erfasst wird, um auf diese Weise sicherzustellen, dass die von Seiten der Rechnereinheit 12 und der darin implementierten Auswerteeinheit 14 verarbeiteten Daten sowie die darauf beruhende Erzeugung eines Signals $S_K$ zeitsynchron mit dem tatsächlichen Betrieb des Lasers 3 sind.

**Bezugszeichenliste**

| | |
|---|---|
| 1 | Endoskop |
| 2 | Lichtleiter |
| 3 | Laser |
| 4 | Lichtaustrittsapertur |
| 5 | Spülflüssigkeitskanal |
| 6 | Spülflüssigkeitsfördereinheit |
| 7 | Spülflüssigkeitsreservoir |
| 8 | Stein |
| 9 | Moduleinheit |
| 10 | Durchflusssensor |
| 11 | Temperatursensor |
| 12 | Rechnereinheit |
| 13 | Eingabemittel |
| 14 | Auswerteeinheit |
| 15 | Komparatoreinheit |
| 16 | Signaleinheit |
| 17 | Mikrofoneinheit |

**Patentansprüche**

1. System zur Erzeugung einer für einen Benutzer wahrnehmbaren Rückmeldung bei der Durchführung einer laserlichtbasierten Lithotripsie, bei der eine Endoskopanordnung zum Einsatz kommt, die einen Arbeitskanal für einen Lichtleiter, der proximalseitig mit einem Laser optisch gekoppelt ist und distalseitig eine Lichtaustrittsapertur aufweist, sowie einen Spülflüssigkeitskanal umfasst, der im Bereich der Lichtaustrittsapertur distalseitig offen mündet und proximalseitig mit einem Spülflüssigkeitsreservoir fluidisch verbunden ist,
   **dadurch gekennzeichnet, dass** das System folgende Komponenten umfasst:

   einen Durchflusssenor, der kontaktfrei zur Spülflüssigkeit eine Spülflussrate bestimmt,
   ein Eingabemittel, über das Betriebsparameter des Lasers, auf deren Grundlage eine am Ort der Lichtaustrittapertur applizierbare Laserleistung ermittelbar ist, einer mit dem Eingabemittel verbundenen Rechnereinheit übermittelbar sind,
   einen Temperatursensor, der kontaktfrei zur Spülflüssigkeit eine Temperatur der Spülflüssigkeit bestimmt,
   eine in der Rechnereinheit implementierte Auswerteeinheit, die zumindest auf der Grundlage der Spülflussrate, der Temperatur der Spülflüssigkeit (Ts) sowie der ermittelten applizierten Laserleistung eine sich während der laserlichtbasierten Lithotripsie intrakorporal am Ort der Lichtaustrittapertur ausbildende Temperatur T numerisch ermittelt, sowie
   eine Komparatoreinheit, die die ermittelte Temperatur T mit einem Schwellwert vergleicht und bei Überschreiten des Schwellwertes die für den Benutzer wahrnehmbare Rückmeldung erzeugt.

2. System nach Anspruch 1,
   **dadurch gekennzeichnet, dass** der Durchflusssensor in Art eines Ultraschallsensors ausgebildet ist.

3. System nach Anspruch 1 oder 2,
   **dadurch gekennzeichnet, dass** das System so konfiguriert ist, dass es je nach chirurgischer Anatomie ausgewählte Betriebsmodi für die Ausführung der auf Laserlicht basierenden Lithotripsie bereitstellt.

4. System nach einem der Ansprüche 1 bis 3,
   **dadurch gekennzeichnet, dass** die Betriebsparameter des Lasers die folgenden vorgebbaren Parameter sind: Laserpulsfrequenz, Einzelpulsenergie,

**5.** System nach einem der Ansprüche 1 bis 4,
**dadurch gekennzeichnet, dass** die Auswerteeinheit auf der Grundlage des folgenden Algorithmus die sich am Ort der Lichtaustrittapertur ausbildende Temperatur T bestimmt:

$$T = \left( 14{,}4\ K * \frac{[Laserleistung\ [W]]}{\left[Sp\ddot{u}lflussrate\ \left[\frac{ml}{min}\right]\right]} \right) + Temp.\,d.\,Sp\ddot{u}lfl\ddot{u}ssigkeit\ [K]$$

**6.** System nach einem der Ansprüche 1 bis 5,
**dadurch gekennzeichnet, dass** die für den Benutzer wahrnehmbare Rückmeldung eine haptisch, akustisch und/oder visuell wahrnehmbare Rückmeldung ist

**7.** System nach einem der Ansprüche 1 bis 6,
**dadurch gekennzeichnet, dass** eine Kühleinheit vorgesehen ist, die thermisch an den Spülflüssigkeitskanal und/oder an das Spülfluidreservoir ankoppelt, und
dass die Kühleinheit durch die Komparatoreinheit aktivierbar oder steuerbar ist.

**8.** System nach einem der Ansprüche 1 bis 7,
**dadurch gekennzeichnet, dass** eine Notabschaltung vorgesehen ist, die durch die Komparatoreinheit aktivierbar ist.

**9.** System nach einem der Ansprüche 1 bis 8,
**dadurch gekennzeichnet, dass** der Durchflusssenor und/oder der Temperatursensor als Nachrüst-Kit zur Anbringung an eine im Einsatz befindliche Endoskopanordnung ausgebildet ist, die zur Durchführung laserlichtbasierter Lithotripsie geeignet ist.

**10.** System nach einem der Ansprüche 1 bis 9,
**dadurch gekennzeichnet, dass** der Arbeitskanal und der Spülflüssigkeitskanal als ein einheitlicher Kanal ausgebildet sind, so dass der längs des Arbeitskanals angeordnete Lichtleiter von Spülflüssigkeit unmittelbar umspült ist.

**11.** System nach einem der Ansprüche 1 bis 10,
**dadurch gekennzeichnet, dass** die Endoskopanordnung einen extrakorporalen Bereich besitzt, der während der Durchführung der Lithotripsie außerhalb eines Patienten verbleibt, und
dass der Temperatursensor und der Durchflusssensor der Moduleinheit im extrakorporalen Bereich der Endoskopanordnung längs des Spülflüssigkeitskanals oder einer mit diesem fluidisch verbundenen Zuleitung angebracht sind.

**12.** System nach einem der Ansprüche 1 bis 11,
**dadurch gekennzeichnet, dass** das System eine Einheit zur Erfassung eines Füllstandes der Spülflüssigkeit in dem Spülflüssigkeitsreservoir vorsieht.

**13.** System nach Anspruch 12,
**dadurch gekennzeichnet, dass** die Einheit ein am Spülflüssigkeitsreservoir vorgesehener Sensor und/oder ein in der Recheneinheit implementierter, softwarebasierter Auswertealgorithmus ist, der auf der Grundlage der sensoriell erfassten Spülflussrate und einer gemessenen Zeitdauer, während der ein Spülflüssigkeitsaustrag erfolgt, den Füllstand ermittelt.

**14.** System nach einem der Ansprüche 1 bis 13,
**dadurch gekennzeichnet, dass** das System so konfiguriert ist, dass es eine Zeitspanne bis zur vollständigen Entleerung des Spülflüssigkeitsreservoirs vorhersagt oder ein Warnsignal erzeugt, wenn eine minimale Restmenge der Spülflüssigkeit erreicht ist.

**15.** System nach einem der Ansprüche 1 bis 14,

**dadurch gekennzeichnet, dass** die sensoriell erfasste Temperatur der Spülflüssigkeit (Ts) der Komparatoreinheit oder einer weiteren Komparatoreinheit zuführbar ist, die bei Unter- oder Überschreiten der Temperatur der Spülflüssigkeit (Ts) unter oder über einen kritischen Temperaturwert ein Signal erzeugt.

16. System nach einem der Ansprüche 1 bis 15,
**dadurch gekennzeichnet, dass** der Spülflüssigkeitskanal proximalseitig mit einer elektrisch betreibbaren Spülflüssigkeitsfördereinheit verbunden ist, und
dass die Spülflüssigkeitsfördereinheit ein Mittel vorsieht, durch das die Spülflüssigkeitsrate in Abhängigkeit der sich am Ort der Lichtaustrittapertur ausbildenden Temperatur T und/oder wenigstens eines Betriebsparameters des Lasers regelbar ist.

17. Verfahren zur Erzeugung einer für einen Benutzer wahrnehmbaren Rückmeldung bei der Durchführung einer auf Laserlicht basierenden Lithotripsie an einer chirurgischen Stelle, wobei das Verfahren folgende Verfahrensschritte umfasst:

Bestimmen einer Spülflussrate einer Spülflüssigkeit, die der chirurgischen Stelle zugeführt wird, ohne dass ein Kontakt mit der Spülungsflüssigkeit besteht,
Wählen von Betriebsparametern eines Lasersystems, so dass am Ort der Lichtaustrittsapertur eine Laserleistung applizierbar ist,
Bestimmen einer Temperatur der Spülflüssigkeit ohne Kontakt mit der Spülflüssigkeit,
numerisches Bestimmen einer Temperatur T, die sich intrakorporal am Ort der Lichtaustrittsapertur während der laserlichtbasierten Lithotripsie ausbildet, zumindest teilweise auf der Grundlage der bestimmten Spülflussrate, der bestimmten Temperatur der Spülflüssigkeit (Ts) und der am Ort der Lichtaustrittsapertur applizierten Laserleistung, und
Vergleichen der bestimmten Temperatur T mit einem Schwellwert und Bereitstellen einer für den Benutzer wahrnehmbaren Rückmeldung, wenn der Schwellenwert überschritten wird.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20180055568 A1
- DE 202017102316 U1